# FASCICULE DE BREVET EUROPEEN

(11) **EP 3 267 899 B1**
(45) Date de publication et mention de la délivrance du brevet: **16.10.2019**
(21) Numéro de dépôt: 16715007.7
(22) Date de dépôt: 09.03.2016
(51) Int. Cl.: A61B 17/06, B21G 1/00, C22F 1/00

(54) **PERFECTIONNEMENTS AUX AIGUILLES HYPER ÉLASTIQUES**
VERBESSERUNGEN AN HYPERELASTISCHEN NADELN
IMPROVEMENTS TO HYPER-ELASTIC NEEDLES

(30) Priorité: 11.03.2015 FR 1552001
(43) Date de publication de la demande: 17.01.2018
(73) Titulaire: Soprane, 69616 Villeurbanne (FR)
(72) Inventeur: LOUBENS, Thierry, 69003 Lyon (FR)
(74) Mandataire: Godard, Xavier
(86) Numéro de dépôt international: PCT/FR2016/050536
(87) Numéro de publication internationale: WO 2016/142625

(56) Documents cités:
- EP-A1- 2 781 194
- EP-A2- 0 619 983
- WO-A1-01/56478
- US-A1- 2004 216 816
- US-A1- 2009 304 772
- US-A1- 2014 242 543
- Alan R. Pelton and al.: "The physical metallurgy of Nitinol for medical application", , 31 mai 2003 (2003-05-31), XP055200560, Extrait de l'Internet: URL:http://www.nitinol.com/wp-content/uplo ads/2012/01/120_Pelton_Russell_DiCello_200 3.pdf [extrait le 2015-07-07]
- O'BRIEN B ET AL: "Passivation of nitinol wire for vascular implants-a demonstration of the benefits", BIOMATERIALS, ELSEVIER SCIENCE PUBLISHERS BV., BARKING, GB, vol. 23, no. 8, 15 avril 2002 (2002-04-15) , pages 1739-1748, XP004348186, ISSN: 0142-9612, DOI: 10.1016/S0142-9612(01)00299-X
- Pelton and al.: "Optimization of processing and properties of medical grade nitinol wire", , 1 janvier 2001 (2001-01-01), XP055200568, Extrait de l'Internet: URL:http://www.nitinol.com/wp-content/uplo ads/2012/01/027.pdf [extrait le 2015-07-07]

## Description

La présente invention est relative à des perfectionnements appliqués aux procédés de fabrications des aiguilles chirurgicales fabriquées à partir d'alliages hyper-élastiques qui sont destinées à être utilisées dans les procédures célioscopiques ou endoscopiques.

On connait par le brevet d'invention EP 0529675 du 31 août 1992, une aiguille chirurgicale réalisée dans un alliage à mémoire de forme qui présente un premier état dit « basse-température », et un second état dit « haute-température ».

L'aiguille dans son état basse-température peut être configurée en une forme allongée afin de permettre son passage dans un tube droit.

L'aiguille dans son état haute-température prend une forme d'arc prédéterminé, et elle est alors adaptée pour être utilisée comme aiguille chirurgicale.

L'aiguille suivant le brevet EP 0529675 est particulièrement adaptée pour des procédures endoscopiques dans lesquelles des éléments sont conduits dans le site chirurgical passant par une canule ou un trocart présentant un diamètre interne de petite dimension.

L'aiguille décrite dans le brevet EP 0529675 présente cependant des inconvénients puisqu'il est nécessaire d'amener le corps de l'aiguille disposée dans le site opératoire à coté d'une source de chaleur pour qu'elle prenne une configuration courbée suivant un arc prédéterminé.

On connait d'après le brevet EP 1251785 appartenant au demandeur une aiguille chirurgicale qui est constituée dans un alliage hyper-élastique présentant, après traitement, deux états distincts permettant, d'une part de contraindre l'aiguille dans une position sensiblement allongée lorsqu'elle est logée dans l'alésage interne d'une canule ou d'un applicateur, et d'autre part lorsqu'elle est extraite de la canule ou de l'applicateur, de pouvoir prendre un profil courbe en arc de cercle du fait de ses propres caractéristiques de super-élasticité ou d'hyper-élasticité.

Le document EP2781194 A1 décrit un procédé de fabrication d'une aiguille chirurgicale dans lequel un fil d'alliage à mémoire de forme est enroulé autour d'un gabarit ayant une forme prédéterminée ou dans lequel l'alliage est chargé dans un moule. Le gabarit ou le moule entier est mis dans un four et est soumis à un traitement thermique (à environ 400°C à 500°C pendant environ une heure).

L'objet de la présente invention est de perfectionner le procédé de fabrication de l'aiguille hyper élastique afin de maintenir les deux états distincts permettant à l'aiguille de passer d'une forme allongée contenue dans la canule à une forme courbe dans le site opératoire tout en garantissant à l'aiguille une résistance à la flexion lors de la perforation des tissus.

Le procédé de fabrication d'une aiguille chirurgicale réalisée à partir d'un fil d'alliage hyper-élastique ou super-élastique comprenant une base de Nickel (Ni), et de Titane (Ti), suivant la présente invention consiste :
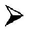 A réaliser une mise en forme à froid ou à température ambiante d'un profil courbe de l'aiguille dans un support approprié ;
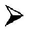 A faire subir un traitement thermique à l'aiguille dans son support afin de mémoriser la géométrie à profil courbe imposée ;
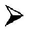 A nettoyer l'aiguille au moyen d'une solution chimique pour retirer la couche d'oxyde déposée sur l'aiguille lors de son traitement thermique.

Dans le procédé de fabrication suivant la présente invention on fait subir à l'aiguille dans son support un recuit dont la température d'échauffement est de 470°C degrés pendant 15 minutes suivi d'un refroidissement à l'eau glacé afin de mémoriser la géométrie à profil courbe imposée .

Dans le procédé de fabrication suivant la présente invention la température de l'eau glacée est comprise en 3 et 5°C degrés.

Dans le procédé de fabrication suivant un mode de réalisation avantageux de la présente invention la solution chimique de nettoyage est constituée de HF+HNO₃+H₂O₂ dans les proportions respectives de 1 :3 :6.

Dans le procédé de fabrication suivant un mode de réalisation avantageux de la présente invention le temps de l'attaque chimique pour le nettoyage de l'aiguille par la solution est compris entre 1 à 3 minutes.

Dans le procédé de fabrication suivant un mode de réalisation avantageux de la présente invention l'aiguille subit préalablement à la mise en forme à froid un traitement thermomécanique permettant d'augmenter sa résistance à la flexion par une augmentation de l'écart entre la température de fin de transformation austénite - martensite et la température d'utilisation.

Dans le procédé de fabrication suivant un mode de réalisation avantageux de la présente invention le profil du fil de l'aiguille est modifié afin que ce dernier présente une section non circulaire.

### DESCRIPTION DE L'INVENTION

Le procédé de fabrication de l'aiguille chirurgicale réalisée à partir d'un fil d'alliage hyper-élastique ou super-élastique comprenant une base de Nickel (Ni) et de Titane (Ti), comprend une première étape de mise en forme à froid ou à température ambiante du profil courbe de l'aiguille dans un support approprié. Cette étape comporte la fixation d'un fil d'alliage à base de Nickel (Ni) et de Titane (Ti) dans un support spécifique permettant d'imposer (à la température ambiante) et de retenir (à des températures élevées) la forme d'une arche d'un rayon de courbure qui correspond au profil désiré de l'aiguille en position d'utilisation dans le site opératoire.

Le tableau ci-dessous résume la géométrie du rayon de courbure devant être imposée aux fils d'alliage de l'aiguille en fonction de son diamètre lors de l'étape de mise en forme :

**Tableau 1**

| Diamètre des fils mm | Rayon de courbure, mm | Déformation |
|---|---|---|
| | | fibre externe, % |
| 1,0± 0.05 | 11,46 | 4.4 |
| 0,6 ± 0.05 | 8,28 | 3. |

Le procédé de fabrication de l'aiguille chirurgicale comporte une seconde étape qui consiste à faire subir un traitement thermique à l'aiguille dans son support afin de mémoriser la géométrie imposée.

Ce traitement thermique permet de mémoriser la géométrie du rayon de courbure imposée lors de la première étape du procédé de fabrication. Le régime de traitement thermique (recuit) est le suivant : 470°C pendant 15 minutes, suivi d'un refroidissement à l'eau glacée à une température d'environ 3 à 5°C.

Le procédé de fabrication de l'aiguille chirurgicale comporte une troisième étape qui consiste à nettoyer l'aiguille au moyen d'un décapage chimique du type polissage électrochimique ou électrolytique ou d'une solution chimique pour retirer la couche d'oxyde déposée sur cette dernière lors de son traitement thermique.

Dans le cas d'un polissage électrolytique, il est prévue d'utiliser un mélange d'acide phosphorique et sulfurique dont les concentrations dépendent des réglages de la densité du courant, de la température du bain d'électrolyte et de la durée du traitement.

Dans le cas de l'utilisation d'une solution chimique cette dernière est constituée principalement d'acide fluorhydrique (HF) et d'acide nitrique (HNO₃) selon la formulation suivante :
HF + HNO₃ + H₂O₂ dans les proportions volumiques respectives de : 1 : 3 : 6.

La durée de l'attaque chimique peut varier entre 1 et 3 minutes en fonction des concentrations chimiques de chaque composante de la solution.

Le procédé de fabrication suivant la présente invention a été validé expérimentalement en utilisant la méthodologie suivante :
- le fil d'alliage pour chaque diamètre indiqué dans le tableau 1 ci-dessus est mis en forme avec les rayons de courbure différents,
- après le traitement thermique, chaque échantillon est inséré dans un cathéter en position allongée ou droite et ensuite déployé pour prendre sa configuration selon le rayon de courbure déterminé,
- cette opération a été répétée de cinq à dix fois et a été suivie par une évaluation de la forme restituée.

Cette expérimentation a permis de vérifier que le stockage de l'aiguille dans un cathéter en position allongée ne perturbe pas son comportement super élastique et qu'aucune déformation plastique irréversible n'est crée dans l'aiguille.

Selon une variante, le procédé de fabrication suivant la présente invention peut comporter une étape de traitements thermomécaniques permettant d'améliorer la résistance à la flexion des aiguilles afin de garantir une stabilité de la position de cette dernière lors de la perforation des tissus.

Pour cela, il est nécessaire de modifier au préalable et avant la mise en forme à froid de l'aiguille les propriétés fonctionnelles des matériaux à mémoire de forme de type hyper-élastique ou super-élastique comprenant une base de Nickel (Ni) et de Titane (Ti) en ajustant les régimes des traitements thermomécaniques.

Pour un alliage donné, la résistance des aiguilles et notamment la résistance à la flexion peut être augmentée si l'écart entre la température de fin de transformation martensitique et la température d'utilisation devient plus important.

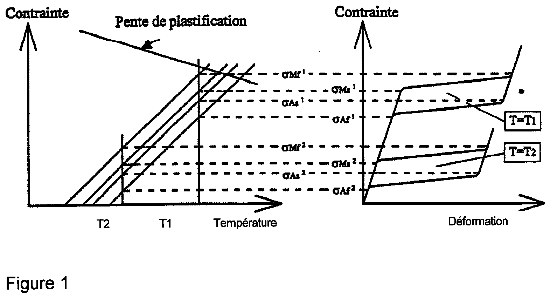

La figure 1 montre l'influence de cet écart entre la température de fin de transformation martensitique et la température d'utilisation.

Le procédé de fabrication suivant l'invention prévoit un traitement thermomécanique de l'alliage utilisé par l'application d'une contrainte variable 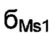 ou 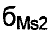 maintenu à une température constante T₁ ou T₂ afin d'augmenter l'écart entre la température de fin de transformation martensitique et la température d'utilisation (figure 1).

Au début, le fil d'alliage formant l'aiguille est complètement austénitique et la contrainte est augmentée jusqu'à ce que la zone de transformation soit traversée. La contrainte est ensuite relâchée pour revenir à l'état initial.

Lorsque la contrainte passe de 0 à 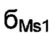 ou 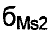 la relation est linéaire et le comportement correspond à celui de l'austénite élastique caractérisée par son module de Young.

Lorsque la contrainte varie entre 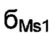 et 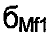 ou 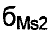 et 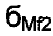 , on entre dans la région de transformation où on constate une "rigidité apparente" (pente dans la zone de transformation) inférieure au module de Young et c'est à ce moment que la transformation de phase s'effectue.

Enfin, lorsque la contrainte devient supérieure à 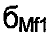 ou 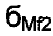 le régime élastique de la martensite orientée est retrouvé. Quand la contrainte est relâchée, les mêmes étapes se reproduisent dans un ordre inverse à des niveaux inférieurs de contrainte (entre 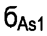 et 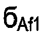 ou 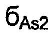 et 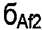 ).

On constate que la transformation de phase s'effectue à des niveaux de contrainte plus élevés au fur et à mesure que l'écart entre ces températures augmente.

On note que la courbe super élastique obtenue à une température **T₁** correspond à un niveau de contrainte plus élevée et donc manifeste une résistance plus importante que celle à une température inférieure T₂.

Des précautions doivent être prises afin d'éviter que la contrainte induite dans le matériau lors de sa déformation n'excède pas la contrainte de début de déformation plastique conventionnelle. Si cette dernière contrainte est dépassée, la plastification du matériau causera une perte définitive des propriétés super élastiques.

Cette augmentation de l'écart entre la température de fin de transformation martensitique et la température d'utilisation permet une augmentation de la résistance à la flexion de l'aiguille.

Selon une autre variante, le procédé de fabrication suivant la présente invention peut comporter une étape consistant à modifier le profil du fil de l'aiguille permettant d'améliorer la résistance à la flexion des aiguilles afin de garantir une stabilité de la position de cette dernière lors de la perforation de tissus.

Pour garantir un bon fonctionnement d'une aiguille de suture, il faut que la flexion dans le plan de la courbure initiale soit privilégiée par rapport aux autres plans de flexion. Cette condition s'avère très important du point de vue de la qualité de la suture parce qu'elle permet de garantir la stabilité de la position de l'aiguille lors de la perforation des tissus, principalement lorsque la force s'exerçant sur l'extrémité de l'aiguille ne demeure pas exactement dans le plan de sa fibre moyenne.

Pour satisfaire cette condition de non-déviation du corps de l'aiguille, il est nécessaire de remplacer la section circulaire de cette dernière par une section non circulaire ayant un moment d'inertie par rapport à l'axe perpendiculaire Y à la force de suture inférieur à celui par rapport à l'axe parallèle Z à celui de la force.

Plus la différence est importante entre les moments d'inertie par rapport aux axes Y et Z meilleure est la garantie que la flexion de l'aiguille se fera dans son plan de courbure initiale, même si la force de suture est légèrement inclinée par rapport à ce même plan.

Le choix d'une section non circulaire présente donc un net avantage par rapport à une section circulaire parce qu'il devient possible de garantir une non-déviation de l'aiguille dans le cas de légères variations de sa position par rapport à un point de suture.

Le procédé de fabrication d'une aiguille chirurgicale réalisée à partir d'un fil d'alliage hyper-élastique ou super-élastique comprenant une base de Nickel (Ni), et de Titane (Ti), selon l'invention ne se limite pas aux applications qui viennent d'être décrites et il doit être entendu que la description qui précède n'a été donnée qu'à titre d'exemple et qu'elle ne limite nullement le domaine de l'invention dont on ne sortirait pas en remplaçant les détails d'exécution décrits par tout autre équivalent.

## Revendications

1. Procédé de fabrication d'une aiguille chirurgicale réalisée à partir d'un fil d'alliage hyper-élastique ou super-élastique comprenant une base de Nickel (Ni) et de Titane (Ti), comprenant une première étape qui consiste :
- à réaliser une mise en forme à froid ou à température ambiante d'un profil courbe de l'aiguille dans un support approprié ;
**caractérisé en ce qu'il comporte**
- une seconde étape qui consiste à faire subir un traitement thermique à l'aiguille dans son support au moyen d'un recuit dont la température d'échauffement est de 470°C pendant 15 minutes suivi d'un refroidissement à l'eau glacée dont la température est comprise entre 3° et 5°C, afin de mémoriser la géométrie à profil courbe imposée;
- et une troisième étape qui consiste à nettoyer l'aiguille au moyen d'un décapage chimique du type polissage électrochimique ou électrolytique ou d'une solution chimique pour retirer la couche d'oxyde déposée sur l'aiguille lors de son traitement thermique.

2. Procédé de fabrication d'une aiguille chirurgicale suivant la revendication 1, **caractérisé en ce que** la solution chimique de nettoyage est constituée de HF+HNO₃+H₂O₂ dans les proportions volumiques respectives de 1 :3 :6.

3. Procédé de fabrication d'une aiguille chirurgicale suivant les revendications 1 et 2 **caractérisé en ce que** le temps de l'attaque chimique pour le nettoyage de l'aiguille par la solution est compris entre 1 à 3 minutes.

4. Procédé de fabrication d'une aiguille chirurgicale suivant la revendication 1, **caractérisé en ce qu'**il consiste à faire subir à l'aiguille, préalablement à la mise en forme à froid, un traitement thermomécanique permettant d'augmenter sa résistance à la flexion par une augmentation de l'écart entre la température de fin de transformation austénite - martensite et la température d'utilisation.

5. Procédé de fabrication d'une aiguille chirurgicale suivant la revendication 1, **caractérisé en ce qu'**il consiste à modifier le profil du fil de l'aiguille afin que ce dernier présente une section non circulaire.

## Patentansprüche

1. Verfahren zur Herstellung einer chirurgischen Nadel, die aus einem hyperelastischen oder superelastischen Legierungsdraht erzeugt wird, umfassend eine Basis aus Nickel (Ni) und aus Titan (Ti), umfassend einen ersten Schritt, der besteht aus:
- dem Durchführen eines Kalt- oder Umgebungstemperator-Formens eines gekrümmten Profils einer Nadel in einem geeigneten Träger;
**dadurch gekennzeichnet, dass** dieses umfasst:
- einen zweiten Schritt, der besteht aus:
- dem Aussetzen der Nadel in ihrem Träger an eine thermische Behandlung mittels eines Glühens, dessen Erhitzungstemperatur 470 °C während 15 Minuten beträgt, gefolgt von einem Abkühlen mit Eiswasser, dessen Temperatur zwischen 3 °C und 5 °C beträgt, um die verliehene Geometrie mit gekrümmtem Profil beizubehalten;
- und einen dritten Schritt, der besteht aus:
- dem Reinigen der Nadel mittels eines chemischen Ätzens vom Typ eines elektrochemischen oder elektrolytischen Polierens oder einer chemischen Lösung, um die Oxidschicht abzuziehen, die auf der Nadel während ihrer thermischen Behandlung abgeschieden wurde.

2. Verfahren zur Herstellung einer chirurgischen Nadel nach Anspruch 1,
**dadurch gekennzeichnet, dass** die chemische Reinigungslösung aus HF + HNO₃ + H₂O₂ in jeweiligen Volumenanteilen von 1 : 3 : 6 besteht.

3. Verfahren zur Herstellung einer chirurgischen Nadel nach den Ansprüche 1 und 2,
**dadurch gekennzeichnet, dass** die Zeit des chemischen Ätzens zur Reinigung der Nadel durch die Lösung zwischen 1 bis 3 Minuten liegt.

4. Verfahren zur Herstellung einer chirurgischen Nadel nach Anspruch 1,
**dadurch gekennzeichnet, dass** dieses daraus besteht, dass die Nadel, vor dem Kalt-Formen, einer thermomechanischen Behandlung ausgesetzt wird, die eine Erhöhung ihrer Biegefestigkeit durch eine Erhöhung der Differenz zwischen der Temperatur am Ende der Austenit-Martensit-Transformation und der Verwendungstemperatur gestattet.

5. Verfahren zur Herstellung einer chirurgischen Nadel nach Anspruch 1,
**dadurch gekennzeichnet, dass** dieses daraus besteht, dass das Profil des Legierungsdrahts modifiziert wird, damit dieser Letztere einen nicht kreisförmigen Querschnitt aufweist.

## Claims

1. Method for manufacturing a surgical needle produced from a wire made of a hyperelastic or superelastic alloy including a nickel (Ni) and titanium (Ti) base, comprising a first step which consists in :
carrying out a cold shaping or a shaping at ambient temperature of a curved profile of the needle in an appropriate support;
**characterized in that it comprises**
- a second step which consists in
subjecting the needle in its support to a thermal treatment by means of an annealing at a heating temperature of 470 °C for 15 minutes followed by cooling with ice water, the temperature of which is between 3 and 5 °C, in order to memorize the imposed curved- profile geometry;
- and a third second step which consists in
cleaning the needle by means of a chemical stripping of the electrochemical or electrolytic polishing type or by means of a chemical solution in order to remove the layer of oxide deposited on the needle during its thermal treatment.

2. Method for manufacturing a surgical needle according to claim 1, **characterized in that** the chemical cleaning solution consists of HF + HNO₃ + H₂O₂ in the respective proportions by volume of 1:3:6.

3. Method for manufacturing a surgical needle according to claims 1 and 2, **characterized in that** the duration of the chemical attack for the cleaning of the needle with the solution is between 1 and 3 minutes.

4. Method for manufacturing a surgical needle according to claim 1, **characterized in that** it consists in subjecting the needle, before the cold shaping, to a thermomechanical treatment making it possible to increase its resistance to bending by increasing the difference between the temperature at the end of the austenite-martensite transformation and the temperature of use.

5. Method for manufacturing a surgical needle according to claim 1, **characterized in that** it consists in modifying the profile of the wire of the needle so that the latter has a non-circular cross-section.
